**Europäisches Patentamt**

**European Patent Office**

**Office européen des brevets**

(19)

(11) Veröffentlichungsnummer : **0 379 082 B1**

(12) # EUROPÄISCHE PATENTSCHRIFT

(45) Veröffentlichungstag der Patentschrift :
**04.08.93 Patentblatt 93/31**

(51) Int. Cl.$^5$ : **A61K 7/11**

(21) Anmeldenummer : **90100612.2**

(22) Anmeldetag : **12.01.90**

(54) **Haarfestigungsmittel.**

(30) Priorität : **18.01.89 DE 3901325**

(43) Veröffentlichungstag der Anmeldung :
**25.07.90 Patentblatt 90/30**

(45) Bekanntmachung des Hinweises auf die
Patenterteilung :
**04.08.93 Patentblatt 93/31**

(84) Benannte Vertragsstaaten :
**DE ES FR GB IT NL**

(56) Entgegenhaltungen :
**EP-A- 0 257 444**
**DE-A- 1 911 306**
**DE-A- 3 627 969**
**FR-A- 2 424 738**

(73) Patentinhaber : **BASF Aktiengesellschaft**
**Carl-Bosch-Strasse 38**
**W-6700 Ludwigshafen (DE)**

(72) Erfinder : **Potthoff-Karl, Birgit Dr.**
**Grundelbachstrasse 112 e**
**W-6940 Weinheim (DE)**
Erfinder : **Sanner, Axel Dr.**
**Lorscher Ring 2c**
**W-6710 Frankenthal (DE)**
Erfinder : **Sperling-Vietmeier, Karin Dr.**
**Im Kirchenstueck 12**
**W-6730 Neustadt (DE)**

EP 0 379 082 B1

Jouve, 18, rue Saint-Denis, 75001 PARIS

## Beschreibung

Die vorliegende Erfindung betrifft Haarfestigungsmittel, welche als Filmbildner Copolymerisate auf der Basis von tert.-Butylacrylat und/oder tert.-Butylmethacrylat mit einem K-Wert von 10 bis 50 enthalten, die durch radikalische Polymerisation von

A) 75 bis 99 Gew.% tert.-Butylacrylat und/oder tert.-Butylmethacrylat als Monomerem A,

B) 1 bis 25 Gew.% Acrylsäure und/oder Methacrylsäure als Monomerem B und

C) 0 bis 10 Gew.% eines weiteren radikalisch copolymerisierbaren Monomeren C

erhältlich sind, wobei die Carboxylgruppen der Copolymerisate teilweise oder vollständig durch Amine neutralisiert sind.

Die japanische Patentschrift 71/27 480 betrifft allgemein Haarspayzubereitungen, die ein Copolymerisat aus einer ungesättigten Carbonsäure und einer zweiten ethylenisch ungesättigten Verbindung in einer mit einem Amin neutralisierten Form enthalten. Als Beispiel wird dort ein Terpolymer aus 45 Gew.% Butylacrylat, 40 Gew.% Methylmethacrylat und 15 Gew.% Acrylsäure genannt. Tertiäre Butylester von ungesättigten Carbonsäuren werden als Ausgangsstoffe für die Herstellung dieser Copolymerisate nicht erwähnt.

In der US-Patentschrift 4 543 249 wird ein Copolymerisat aus 70 bis 90 Gew.% Methylmethacrylat und 10 bis 30 Gew.% Methacrylsäure als Bestandteil einer Haarsprayzubereitung beschrieben, dessen Carboxylgruppen zu 50 bis 100 % mit einer wasserlöslichen Base neutralisiert sind.

Die US-Patentschrift 1 410 012 betrifft Haarsprayzubereitungen, die ein nicht neutralisiertes Copolymerisat aus 10 bis 90 Gew.% Acrylsäure oder Methacrylsäure und 10 bis 90 Gew.% eines $C_1$-$C_3$-Alkylacrylates oder -methacrylates enthalten.

Die Patentschrift EP-A-0257 444 beschreibt ein 3-Komponenten-Polymerisat mit mindestens 20 Gew % Vinylpyrrolidon als dritte Monomere.

Für die Haarkosmetik werden in zunehmendem Maße Sprayzubereitungen mit Kohlenwasserstoffen anstelle von halogenierten Kohlenwasserstoffen als Treibmittel eingesetzt. Die hierbei als Filmbildner verwendeten, zum oben angeführten Stand der Technik gehörigen Copolymerisate aus Acryl- oder Methacrylsäure und deren Alkylestern zeigen, auch in der neutralisierten Form, noch teilweise verbesserungsbedürftige Werte für die Verträglichkeit mit den unpolaren Kohlenwasserstoffen der Sprayzubereitungen, d.h. die Löslichkeit in ihnen ist noch nicht hoch genug. Außerdem läßt oft die haarfestigende Wirkung dieser Copolymerisate noch zu wünschen übrig.

Aufgabe der vorliegenden Erfindung war es, einen Filmbildner für die Haarkosmetik bereitzustellen, der sich durch eine gute Verträglichkeit mit unpolaren Treibmitteln auf der Basis von Kohlenwasserstoffen auszeichnet und gleichzeitig eine gute haarfestigende Wirkung zeigt.

Demgemäß wurden die eingangs definierten Haarfestigungsmittel gefunden.

Die in den erfindungsgemäßen Haarfestigungsmitteln enthaltenen Copolymerisate sind hauptsächlich aus 75 bis 99 Gew.%, vorzugsweise 85 bis 98 Gew.% tert.-Butylacrylat und/oder tert.-Butylmethacrylat als Monomerem A und 1 bis 25 Gew.%, vorzugsweise 2 bis 15 Gew.% Acrylsäure oder Methacrylsäure als Monomerem B aufgebaut. Besonders gute Ergebnisse erzielt man mit einem Copolymerisat aus 75 bis 99 Gew.%, vorzugsweise 85 bis 98 Gew.% tert.-Butylacrylat und 1 bis 25 Gew.%, vorzugsweise 2 bis 15 Gew.% Methacrylsäure.

Zur geringfügigen Abänderung der Eigenschaften des Copolymerisates kann ein weiteres radikalisch copolymerisierbares Monomeres C in einer Menge bis zu 10 Gew.%, insbesondere bis zu 5 Gew.%, mitenthalten sein. Als Monomere C dienen z.B. der Methyl-, Ethyl-, n-Propyl- oder n-Butylester der Acryl- oder Methacrylsäure, N-Vinylpyrrolidon und Vinylacetat.

Diese Copolymerisate werden durch radikalische Copolymerisation der Monomeren A, B und gegebenenfalls C hergestellt. Hierbei arbeitet man nach den üblichen Polymerisationstechniken, zum Beispiel nach den Methoden der Suspensions-, Emulsions- oder Lösungspolymerisation.

Als besonders zweckmäßig hat sich die Lösungspolymerisation in einem organischen Lösungsmittel, in der Regel einem Alkohol, herausgestellt. Man arbeitet hier üblicherweise bei Temperaturen von 60 bis 130°C, wobei die Umsetzung bei Normaldruck oder unter Eigendruck durchgeführt werden kann.

Als Initiatoren für die radikalisch ablaufende Polymerisationsreaktion werden die üblichen Peroxo- oder Azoverbindungen, beispielsweise Dibenzoylperoxid, tert.-Butylperpivalat, tert.-Butylper-2-ethylhexanoat, Ditert.-butylperoxid, tert.-Butylhydroperoxid, 2,5-Dimethyl-2,5-di(tert.-butylperoxy)hexan oder Azo-bis-isobutyronitril, zweckmäßigerweise in Mengen von 0,1 bis 2 Gew.%, bezogen auf das Gewicht der Monomeren, eingesetzt. Man wählt die Mengen an Monomeren und Lösungsmittel zweckmäßigerweise so, daß man 30 bis 80 gew.%ige Lösungen der Copolymerisate erhält.

Die Copolymerisate sollen K-Werte von 10 bis 50, vorzugsweise 15 bis 35, aufweisen. Der jeweils gewünschte K-Wert läßt sich in an sich bekannter Weise durch Wahl der Polymerisationsbedingungen, beispiels-

2

weise der Polymerisationsdauer und der Initiatorkonzentration, einstellen. Die K-Werte werden nach Fikentscher, Cellulosechemie, Bd. 13, S. 58 bis 64 (1932) bei 25°C in ethanolischer Lösung gemessen und stellen ein Maß für das Molgewicht dar.

Derartige Copolymerisate haben üblicherweise Glasübergangstemperaturen zwischen 50 und 130°C, insbesondere zwischen 60 und 100°C.

Für die Verwendung in den erfindungsgemäßen Haarfestigungsmitteln werden die Carboxylgruppen der so erhaltenen Copolymerisate mit einem Amin teilweise oder vollständig, zweckmäßigerweise zu 5 bis 100 %, vorzugsweise zu 30 bis 90 %, neutralisiert. Die Neutralisation erfolgt bevorzugt mit

- einem Mono-, Di- oder Trialkanolamin mit 2 bis 5 C-Atomen im Alkanolrest, der gegebenenfalls in veretherter Form vorliegt, beispielsweise Mono-, Di- und Triethanolamin, Mono-, Di- und Tri-n-propanolamin, Mono-, Di- und Triisopropanolamin, 2-Amino-2-methylpropanol und Di(2-methoxyethyl)amin,
- einem Alkandiolamin mit 2 bis 5 C-Atomen, beispielsweise 2-Amino-2-methylpropan-1,3-diol und 2-Amino-2-ethylpropan-1,3-diol, oder
- einem primären, sekundären oder tertiären Alkylamin mit insgesamt 5 bis 10 C-Atomen, beispielsweise N,N-Diethylpropylamin.

Besonders gute Ergebnisse erzielt man mit 2-Amino-2-methylpropanol, Triisopropanolamin und 2-Amino-2-ethylpropan-1,3-diol.

Die erfindungsgemäßen Haarfestigungsmittel kommen beispielsweise als Haargele, Haarschäume und vor allem als Frisurenfestiger in Form von Sprayzubereitungen zur Anwendung. Besonders bevorzugt werden Haarsprayzubereitungen, die die folgenden Bestandteile enthalten:

- 0,1 bis 20 Gew.%, vorzugsweise 0,5 bis 10 Gew.%, insbesondere 2 bis 10 Gew.% des teilweise oder vollständig neutralisierten Copolymerisates
- 10 bis 95 Gew.%, vorzugsweise 20 bis 60 Gew.%, insbesondere 25 bis 50 Gew.% eines üblichen Lösungsmittels wie vor allem Ethanol und Isopropanol und daneben auch Aceton, n-Propanol, n-Butanol, 2-Methoxypropan-1-ol, n-Pentan, n-Hexan, Cyclohexan, n-Heptan, n-Octan oder Dichlormethan oder deren Gemische
- 5 bis 90 Gew.%, vorzugsweise 30 bis 80 Gew.%, insbesondere 45 bis 70 Gew.% eines üblichen Treibmittels wie Propan, n-Butan, Isobutan, 2,2-Dimethylbutan, Isopentan, Dimethylether, Fluortrichlormethan, Dichlordifluormethan oder Dichlortetrafluorethan oder deren Gemische. Als Treibmittel (Treibgase) kommen von den genannten Verbindungen vor allem die Kohlenwasserstoffe und insbesondere Propan und n-Butan zur Anwendung. Gegebenenfalls werden einer oder mehrere der genannten Fluorchlorkohlenwasserstoffe in Treibmittelmischungen mitverwendet, jedoch nur in geringen Mengen, etwa bis zu 20 Gew.%, bezogen auf die Treibmittelmischung.

Außerdem können diese Sprayzubereitungen noch geringe Mengen an Parfümölen, beispielsweise 0,1 bis 5,0 Gew.% , enthalten.

Eine Standard-Sprayformulierung weist beispielsweise die folgende Zusammensetzung auf:

6,3 Gew.%    des zu 75 % mit 2-Amino-2-methylpropanol neutralisierten Copolymerisates
33,7 Gew.%   Ethanol
60,0 Gew.%   Propan/n-Butan im Gewichtsverhältnis von 40:60.

Ein überwiegender Teil des Ethanols kann durch ein anderes Lösungsmittel, beispielsweise einen Kohlenwasserstoff wie n-Pentan oder n-Hexan, ersetzt werden, ohne daß sich die haarfestigende Wirkung verschlechtert. Ebenso kann der Anteil des neutralisierten Copolymerisates auf 8 bis 9 Gew.% erhöht werden. Als Treibmittel kann auch n-Butan alleine verwendet werden.

Die in den erfindungsgemäßen Haarfestigungsmitteln enthaltenen Copolymerisate zeichnen sich durch ihre hohe Verträglichkeit mit den unpolaren Treibmitteln in Sprayzubereitungen, insbesondere mit Kohlenwasserstoffen wie Propan oder n-Butan oder ihrem Gemisch aus. In der Regel erreicht man mit ihnen Verträglichkeitswerte zwischen 70 und 85 Gew.% bei einer gleichzeitigen außergewöhnlich guten haarfestigenden Wirkung, ersichtlich an den hohen Werten für die Curl-Retention, die hierbei normalerweise über 90 % liegen. Außerdem zeichnen sich die erfindungsgemäßen Haarfestigungsmittel dadurch aus, daß sie das Haar praktisch nicht verkleben.

Beispiele

Beispiel 1

Copolymerisat aus 92 Gew.% tert.-Butylacrylat und 8 Gew.% Methacrylsäure

Eine Lösung von 92 g tert.-Butylacrylat, 8 g Methacrylsäure und 1,0 g tert.-Butylperpivalat in 545 g Ethanol wurde auf 75°C erwärmt. Nach dem Anspringen der Polymerisation, erkennbar an einer Viskositätserhöhung, wurden gleichzeitig eine Mischung aus 828 g tert.-Butylacrylat, 72 g Methacrylsäure und 88 g Ethanol und eine Lösung von 4,9 g tert.-Butylperpivalat in 123 g Ethanol in Laufe von 3 Stunden zugegeben, wobei die Temperatur bei schwachem Sieden auf 77 bis 80°C gehalten wurde. Anschließend wurde bei der gleichen Temperatur eine Lösung von 4,9 g tert.-Butylperpivalat in 123 g Ethanol innerhalb von weiteren 3 Stunden zugetropft.

Der Polymerisatgehalt der erhaltenen Lösung betrug 53 Gew.%. Das Copolymerisat hatte einen K-Wert von 22,8 (gemessen in 2,0 gew.%iger ethanolischer Lösung bei 25°C) und eine Glasübergangstemperatur von 69°C.

Beispiele 2 bis 7

Die Beispiele 2 bis 4 (siehe Tabelle) betreffen Copolymerisate aus tert.-Butylacrylat und Methacrylsäure unterschiedlicher Zusammensetzung. In den Vergleichsbeispielen 5 bis 7 (siehe Tabelle) werden Copolymerisate aus n-Butylmethacrylat oder (2-Ethylhexyl)acrylat und Methacrylsäure beschrieben. Die Copolymerisate 2 bis 7 wurden analog Beispiel 1 hergestellt.

Eigenschaften der Copolymerisate

Die folgende Tabelle zeigt die Werte für die Zusammensetzung, Kohlenwasserstoffverträglichkeit und die haarfestigende Wirkung dieser Copolymerisate. Außerdem wird die Klebrigkeit der Filme auf dem Haar beurteilt, das mit den entsprechenden Haarsprayformulierungen behandelt wurde.

Tabelle:
Zusammensetzung, Kohlenwasserstoffverträglichkeit, Curl-Retention und Klebrigkeit der Filme auf dem Haar

| Bei-spiel | Zusammensetzung [Gew.%] | | | | Kohlenwasser-stoffverträg-lichkeit mit Propan/n-Butan (40:60) [Gew.%] | Curl-Retention [%] | Klebrig-keit |
|---|---|---|---|---|---|---|---|
| | tBA | BMA | EHA | MAS | | | |
| 1 | 92 | | | 8 | 80 | 94 | keine |
| 2 | 87 | | | 13 | 70 | 91 | keine |
| 3 | 90 | | | 10 | 71 | 92 | keine |
| 4 | 95 | | | 5 | 82 | 91 | keine |
| Vgl.-Bsp: | | | | | | | |
| 5 | | 90 | | 10 | 67 | 50 | deutliche |
| 6 | | 95 | | 5 | 74 | 47 | deutliche |
| 7 | | | 90 | 10 | 94 | 45 | deutliche |

tBA: tert.-Butylacrylat,
BMA: n-Butylmethacrylat,
EHA: (2-Ethylhexyl)acrylat
MAS: Methacrylsäure

Der Wert für die Kohlenwasserstoffverträglichkeit mit einer Propan/n-Butan-Mischung im Gewichtsverhältnis von 40:60 gibt an, wieviel Gew.% dieses Treibgasgemisches eine Sprayzubereitung, die neben Ethanol als Lösungsmittel 3 Gew.% des neutralisierten Copolymeren aufweist, maximal enthalten darf, ohne daß bei 0°C eine Trübung auftritt.

Die Curl-Retention ist ein Maß für die haarfestigende Wirkung. Sie wird im Modellversuch an Haarlocken gemessen, die durch eine übliche Wasserwelle an ca. 15 cm langen Haaren erzeugt und mit der jeweiligen Sprayzubereitung aus 10 cm Entfernung 4 sec lang besprüht worden sind. Nach einer Verweilzeit von 5 Stunden der aufgehängten Locken in einer Klimakammer bei 25°C und 90 % relativer Luftfeuchtigkeit wird die relative Verformung (Aufweitung) der Locken, bezogen auf ihre ursprüngliche Form, bestimmt. Ein hoher Wert bedeutet eine hohe Festigungswirkung, d.h. bei 100 % bliebe die ursprüngliche Form vollständig erhalten.

Die Curl-Retention und die Klebrigkeit für die Copolymerisate der Beispiele 1 bis 4 und der Vergleichsbeispiele 5 bis 7 wurde jeweils mit der folgenden Standard-Sprayformulierung bestimmt:

6,3 Gew.%    Copolymerisat, welches zu 75 % mit 2-Amino-2-methylpropanol neutralisiert worden war
33,7 Gew.%   Ethanol
60,0 Gew.%   Propan/n-Butan (40:60)

## Patentansprüche

### Patentansprüche für folgende Vertragsstaaten : DE, FR, GB, IT, NL

1.  Haarfestigungsmittel, enthaltend als Filmbildner Copolymerisate auf der Basis von tert.-Butylacrylat und/oder tert.-Butylmethacrylat mit einem K-Wert von 10 bis 50, erhältlich durch radikalische Polymerisation von
    A) 75 bis 99 Gew.% tert.-Butylacrylat und/oder tert.-Butylmethacrylat als Monomerem A,
    B) 1 bis 25 Gew.% Acrylsäure und/oder Methacrylsäure als Monomerem B und
    C) 0 bis 10 Gew.% eines weiteren radikalisch copolymerisierbaren Monomeren C,
    wobei die Carboxylgruppen der Copolymerisate teilweise oder vollständig durch Amine neutralisiert sind.

2.  Haarfestigungsmittel nach Anspruch 1, in denen der Filmbildner aus
    A) 85 bis 98 Gew.% tert.-Butylacrylat und/oder tert.-Butylmethacrylat als Monomerem A,
    B) 2 bis 15 Gew.% Acrylsäure und/oder Methacrylsäure als Monomerem B und
    C) 0 bis 10 Gew.% eines weiteren radikalisch copolymerisierbaren Monomeren C
    aufgebaut ist.

3.  Haarfestigungsmittel nach Anspruch 1, in denen der Filmbildner aus
    A) 75 bis 99 Gew.% tert.-Butylacrylat als Monomerem A,
    B) 1 bis 25 Gew.% Methacrylsäure als Monomerem B und
    C) 0 bis 10 Gew.% eines weiteren radikalisch copolymerisierbaren Monomeren C
    aufgebaut ist.

4.  Haarfestigungsmittel nach Anspruch 2, in denen der Filmbildner aus
    A) 85 bis 98 Gew.% tert.-Butylacrylat als Monomerem A,
    B) 2 bis 15 Gew.% Methacrylsäure als Monomerem B und
    C) 0 bis 10 Gew.% eines weiteren radikalisch copolymerisierbaren Monomeren C
    aufgebaut ist.

5.  Haarfestigungsmittel in Form von Sprayzubereitungen, enthaltend neben hierbei üblichen Lösungsmitteln und Treibmitteln 0,1 bis 20 Gew.% eines Copolymerisates gemäß den Ansprüchen 1 bis 4.

6.  Verwendung der Copolymerisate gemäß den Ansprüchen 1 bis 4 als Filmbildner in Haarfestigungsmitteln.

### Patentansprüche für folgenden Vertragsstaat: ES

1.  Verfahren zur Herstellung von Haarfestigungsmitteln, dadurch gekennzeichnet, daß man Copolymerisate auf der Basis von tert.-Butylacrylat und/oder tert.-Butylmethacrylat mit einem K-Wert von 10 bis 50, erhältlich durch radikalische Polymerisation von
    A) 75 bis 99 Gew.% tert.-Butylacrylat und/oder tert.-Butylmethacrylat als Monomerem A,
    B) 1 bis 25 Gew.% Acrylsäure und/oder Methacrylsäure als Monomerem B und
    C) 0 bis 10 Gew.% eines weiteren radikalisch copolymerisierbaren Monomeren C,
    wobei die Carboxylgruppen der Copolymerisate teilweise oder vollständig durch Amine neutralisiert sind, mit den hierbei üblichen Bestandteilen mischt.

2. Verfahren zur Herstellung von Haarfestigungsmitteln nach Anspruch 1, dadurch gekennzeichnet, daß man hierzu Copolymerisate verwendet, die aus

A) 85 bis 98 Gew.% tert.-Butylacrylat und/oder tert.-Butylmethacrylat als Monomerem A,

B) 2 bis 15 Gew.% Acrylsäure und/oder Methacrylsäure als Monomerem B und

C) 0 bis 10 Gew.% eines weiteren radikalisch copolymerisierbaren Monomeren C

aufgebaut sind.

3. Verfahren zur Herstellung von Haarfestigungsmitteln nach Anspruch 1, dadurch gekennzeichnet, daß man hierzu Copolymerisate verwendet, die aus

A) 75 bis 99 Gew.% tert.-Butylacrylat als Monomerem A,

B) 1 bis 25 Gew.% Methacrylsäure als Monomerem B und

C) 0 bis 10 Gew.% eines weiteren radikalisch copolymerisierbaren Monomeren C

aufgebaut sind.

4. Verfahren zur Herstellung von Haarfestigungsmitteln nach Anspruch 2, dadurch gekennzeichnet, daß man hierzu Copolymerisate verwendet, die aus

A) 85 bis 98 Gew.% tert.-Butylacrylat als Monomerem A,

B) 2 bis 15 Gew.% Methacrylsäure als Monomerem B und

C) 0 bis 10 Gew.% eines weiteren radikalisch copolymerisierbaren Monomeren C

aufgebaut sind.

5. Verfahren zur Herstellung von Haarfestigungsmitteln in Form von Sprayzubereitungen, dadurch gekennzeichnet, daß man 0,1 bis 20 Gew.-% eines Copolymerisates gemäß den Ansprüchen 1 bis 4, bezogen auf die Gesamtmenge der Zubereitung, mit den hierbei üblichen Lösungsmitteln und Treibmitteln mischt.

## Claims

### Claims for the following Contracting States : DE, FR, GB, IT, NL

1. A hair setting composition containing as the film former a copolymer based on tert-butyl acrylate or tert-butyl methacrylate, having a K value of from 10 to 50 and obtainable by free radical polymerization of

A) from 75 to 99% by weight of tert-butyl acrylate or tert-butyl methacrylate as monomer A,

B) from 1 to 25% by weight of acrylic acid or methacrylic acid as monomer B and

C) from 0 to 10% by weight of a further free-radically copolymerizable monomer C,

the carboxyl groups of the copolymer having been partly or wholly neutralized by an amine.

2. A hair setting composition as claimed in claim 1, wherein the film former is composed of

A) from 85 to 98% by weight of tert-butyl acrylate or tert-butyl methacrylate as monomer A,

B) from 2 to 15% by weight of acrylic acid or methacrylic acid as monomer B and

C) from 0 to 10% by weight of a further free-radically copolymerizable monomer C.

3. A hair setting composition as claimed in claim 1, wherein the film former is composed of

A) from 75 to 99% by weight of tert-butyl acrylate as monomer A,

B) from 1 to 25% by weight of methacrylic acid as monomer B and

C) from 0 to 10% by weight of a further free-radically copolymerizable monomer C.

4. A hair setting composition as claimed in claim 2, wherein the film former is composed of

A) from 85 to 98% by weight of tert-butyl acrylate as monomer A,

B) from 2 to 15% by weight of methacrylic acid as monomer B and

C) from 0 to 10% by weight of a further free-radically copolymerizable monomer C.

5. A hair setting composition in the form of a spray containing from 0.1 to 20% by weight of a copolymer as claimed in any of claims 1 to 4, as well as customary solvents and propellants.

6. The use of a copolymer as claimed in any of claims 1 to 4 as a film former in hair setting compositions.

**Claims for the following Contracting State : ES**

1. A process for preparing a hair setting composition, which comprises mixing a copolymer based on tert-butyl acrylate or tert-butyl methacrylate, having a K value of from 10 to 50 and obtainable by free radical polymerization of
   A) from 75 to 99% by weight of tert-butyl acrylate or tert-butyl methacrylate as monomer A,
   B) from 1 to 25% by weight of acrylic acid or methacrylic acid as monomer B and
   C) from 0 to 10% by weight of a further free-radically copolymerizable monomer C,
   the carboxyl groups of the copolymer having been partly or wholly neutralized by an amine, with the customary ingredients therefor.

2. A proces for preparing a hair setting composition as claimed in claim 1, wherein the copolymer used is composed of
   A) from 85 to 98% by weight of tert-butyl acrylate or tert-butyl methacrylate as monomer A,
   B) from 2 to 15% by weight of acrylic acid or methacrylic acid as monomer B and
   C) from 0 to 10% by weight of a further free-radically copolymerizable monomer C.

3. A process for the preparation of a hair setting composition as claimed in claim 1, wherein the copolymer used is composed of
   A) from 75 to 99% by weight of tert-butyl acrylate as monomer A,
   B) from 1 to 25% by weight of methacrylic acid as monomer B and
   C) from 0 to 10% by weight of a further free-radically copolymerizable monomer C.

4. A process for the preparation of a hair setting composition as claimed in claim 2, wherein the copolymer used is composed of
   A) from 85 to 98% by weight of tert-butyl acrylate as monomer A,
   B) from 2 to 15% by weight of methacrylic acid as monomer B and
   C) from 0 to 10% by weight of a further free-radically copolymerizable monomer C.

5. A process for preparing a hair setting composition in the form of a spray, which comprises mixing from 0.1 to 20% by weight of a copolymer as claimed in any of claims 1 to 4, based on the total amount of spray, with the customary solvents and propellants therefor.


**Revendications**


**Revendications pour les Etats contractants suivants : DE, FR, GB, IT, NL**

1. Fixateur capillaire contenant, comme filmogènes, des copolymères à base d'acrylate de tert.-butyle et/ou de méthacrylate de tert.-butyle ayant une valeur K de 10 à 50, obtenus par polymérisation radicalaire de
   A) 75 à 99% en poids d'acrylate de tert.-butyle et/ou de méthacrylate de tert.-butyle en tant que monomère A,
   B) 1 à 25% en poids d'acide acrylique et/ou d'acide méthacrylique en tant que monomère B et
   C) 0 à 10% en poids d'un autre monomère C susceptible de copolymérisation radicalaire,
   les groupements carboxyle des copolymères étant neutralisés partiellement ou complètement par des amines.

2. Fixateur capillaire selon la revendication 1, dans lequel le filmogène est composé de
   A) 85 à 98% en poids d'acrylate de tert.-butyle et/ou de méthacrylate de tert.-butyle en tant que monomère A,
   B) 2 à 15% en poids d'acide acrylique et/ou d'acide méthacrylique en tant que monomère B et
   C) 0 à 10% en poids d'un autre monomère C susceptible de copolymérisation radicalaire.

3. Fixateur capillaire selon la revendication 1, dans lequel le filmogène est composé de
   A) 75 à 99% en poids d'acrylate de tert.-butyle en tant que monomère A,
   B) 1 à 25% en poids d'acide méthacrylique en tant que monomère B et
   C) 0 à 10% en poids d'un autre monomère C susceptible de copolymérisation radicalaire.

4. Fixateur capillaire selon la revendication 2, dans lequel le filmogène est composé de

A) 85 à 98% en poids d'acrylate de tert. -butyle en tant que monomère A,

B) 2 à 15% en poids d'acide méthacrylique en tant que monomère B et

C) 0 à 10% en poids d'un autre monomère C susceptible de copolymérisation radicalaire.

5. Fixateurs capillaires sous forme de préparations à pulvériser, contenant, outre des solvants et des agents pulseurs usuels à cet effet, de 0,1 à 20% en poids d'un copolymère selon l'une quelconque des revendications 1 à 4.

6. Utilisation des copolymères selon l'une quelconque des revendications 1 à 4 comme filmogènes dans des fixateurs capillaires.

**Revendications pour l'Etat contractant suivant : ES**

1. Procédé de préparation de fixateurs capillaires, caractérisé en ce qu'on mélange, avec les constituants usuels à cet effet, des copolymères à base d'acrylate de tert.-butyle et/ou de méthacrylate de tert.-butyle ayant une valeur K de 10 à 50, obtenus par polymérisation radicalaire de

A) 75 à 99% en poids d'acrylate de tert. -butyle et/ou de méthacrylate de tert. -butyle en tant que monomère A,

B) 1 à 25% en poids d'acide acrylique et/ou d'acide méthacrylique en tant que monomère B et

C) 0 à 10% en poids d'un autre monomère C susceptible de copolymérisation radicalaire,

les groupements carboxyle des copolymères étant neutralisés partiellement ou complètement par des amines.

2. Procédé de préparation de fixateurs capillaires selon la revendication 1, caractérisé en ce qu'on utilise à cette fin des copolymères qui sont composés de

A) 85 à 98% en poids d'acrylate de tert.-butyle et/ou de méthacrylate de tert.-butyle en tant que monomère A,

B) 2 à 15% en poids d'acide acrylique et/ou d'acide méthacrylique en tant que monomère B et

C) 0 à 10% en poids d'un autre monomère C susceptible de copolymérisation radicalaire,

3. Procédé de préparation de fixateurs capillaires selon la revendication 1, caractérisé en ce qu'on utilise à cette fin des copolymères qui sont composés de

A) 75 à 99% en poids d'acrylate de tert.-butyle en tant que monomère A,

B) 1 à 25% en poids d'acide méthacrylique en tant que monomère B et

C) 0 à 10% en poids d'un autre monomère C susceptible de copolymérisation radicalaire.

4. Procédé de préparation de fixateurs capillaires selon la revendication 2, caractérisé en ce qu'on utilise à cette fin des copolymères qui sont composés de

A) 85 à 98% en poids d'acrylate de tert.-butyle en tant que monomère A,

B) 2 à 15% en poids d'acide méthacrylique en tant que monomère B et

C) 0 à 10% en poids d'un autre monomère C susceptible de copolymérisation radicalaire.

5. Procédé de préparation de fixateurs capillaires sous forme de préparations à pulvériser, caractérisé en ce qu'on mélange de 0,1 à 20% en poids d'un copolymère selon l'une quelconque des revendications 1 à 4, par rapport & la quantité totale de la préparation, avec les solvants et les agents pulseurs usuels à cet effet.